# EUROPEAN PATENT APPLICATION

(11) **EP 4 324 927 A1**
(43) Date of publication of application: **21.02.2024**
(21) Application number: 21936697.8
(22) Date of filing: 13.09.2021
(51) Int. Cl.: C12P 19/30, C12N 9/10, C12N 9/12

(54) **ENZYME COMPOSITION FOR PREPARING ?-NICOTINAMIDE MONONUCLEOTIDE, AND APPLICATION THEREOF**

(30) Priority: 14.04.2021 CN 202110397619
(71) Applicant: Shenzhen Readline Biotech Co., Ltd., Shenzhen, Guangdong 518052 (CN)
(72) Inventor: YU, Tiemei, ShenZhen, Guangdong 518052 (CN); HE, Xiuxiu, ShenZhen, Guangdong 518052 (CN); QIN, Guofu, ShenZhen, Guangdong 518052 (CN); LING, Ruimei, ShenZhen, Guangdong 518052 (CN); LIN, Lifeng, ShenZhen, Guangdong 518052 (CN); TAN, Wenjing, ShenZhen, Guangdong 518052 (CN); PAN, Junfeng, ShenZhen, Guangdong 518052 (CN); LIU, Jian, ShenZhen, Guangdong 518052 (CN)
(74) Representative: Germain Maureau
(86) International application number: PCT/CN2021/117960
(87) International publication number: WO 2022/217827

(57) **Abstract**

The present invention relates to the field of biotechnologies. Disclosed are an enzyme composition for preparing β-nicotinamide mononucleotide (NMN), and an application thereof. In the present invention, by using adenosine and nicotinamide as raw materials, D-ribose l-phosphate and a nicotinamide ribose intermediate are generated under enzyme catalysis of an enzyme composition of a PNP enzyme and an NRK enzyme, and finally NMN is obtained; only two enzymes need to be involved in the whole reaction system, and the by-product adenine can be recycled. Moreover, only one molecule of ATP needs to be consumed for generating one molecule of NMN, thereby greatly reducing the process cost. The reaction of synthesizing NMN by NRK enzyme catalysis in the last step is irreversible, and thus, the substrate conversion rate can be greatly improved and the production cost can be further reduced.

## Description

This application claims the priority of Chinese Patent Application No. 202110397619.0, filed with the China National Intellectual Property Administration on April 14, 2021, and titled with "ENZYME COMPOSITION FOR PREPARING β-NICOTINAMIDE MONONUCLEOTIDE, AND APPLICATION THEREOF", which is hereby incorporated by reference in its entirety.

### FIELD

The present invention relates to the field of biotechnology, specifically to an enzyme composition for preparing β-nicotinamide mononucleotide and uses thereof.

### BACKGROUND

β-Nicotinamide mononucleotide (abbreviated as NMN) is a substance present in organisms. Once adenylated by nicotinamide nucleotide adenosyltransferase, it is converted into nicotinamide adenine dinucleotide (NAD+, also known as coenzyme I), an important substance necessary for the survival of biological cells. A study published in Science by David Scinclair's research team in March 2017 showed that the increase in NAD+ in mice reversed the signs of tissue and muscle aging in older mice, suggesting the possibility of human rejuvenation. Due to its large molecular weight, NAD+ cannot be ingested into cells by oral administration, and instead primarily relies on cellular synthesis with low levels. However, recent research on NMN, a small molecule precursor of NAD+, has discovered that consuming β-NMN can significantly increase NAD+ levels in the body and significantly inhibit age-related metabolism, making β-NMN a "miracle drug of youth". To date, nicotinamide mononucleotide has been demonstrated to have a range of medical and healthcare applications, such as the delay of aging, treatment of senile conditions like Parkinson's disease, regulation of insulin secretion, and modulation of mRNA expression.

At present, the main methods for synthesizing NMN include chemical synthesis and biocatalysis. Among them, chemical methods are high in cost and cause serious environmental pollution, so they have been gradually replaced by biocatalytic methods. In comparison, bioenzymatic catalytic production of NMN is more efficient, cost-effective, and eco-friendly. Three biocatalytic methods are currently being used for NMN production. The first one uses nicotinamide ribose as a raw material and generates NMN under the supply of ATP through nicotinamide riboside kinase (EC 2.7.1.22). The second one uses nicotinamide, ribose and ATP as substrates to generate NMN through a reaction catalyzed by D-ribokinase (EC2.7.1.15), ribose phosphate pyrophosphokinase (EC2.7.6.1), and nicotinamide phosphoribosyltransferase (EC.2.4.2.12). The third method uses adenosine or AMP, ATP, and nicotinamide as raw materials to generate NMN through a reaction catalyzed by adenosine kinase (EC 2.7.1.20) (not required when AMP is used as raw material), adenine phosphoribosyltransferase (EC 2.4.2.7) and nicotinamide phosphoribosyltransferase (EC.2.4.2.12).

The above first method uses nicotinamide ribose directly as a raw material and has a high substrate conversion rate, but nicotinamide ribose is expensive and has no cost advantage. The second and third methods both use PRPP and nicotinamide to prepare NMN through nicotinamide phosphoribosyltransferase (EC.2.4.2.12). Because nicotinamide phosphoribosyltransferase catalyzes a reversible reaction, it can also hydrolyze NMN while synthesizing NMN, leading to a low reaction conversion rate. In addition, the intermediate PRPP compound is unstable and is not conducive to the reaction. Furthermore, both methods require ATP to participate in several reaction steps, resulting in a high consumption of ATP throughout the process, and thus the production cost of this biocatalytic method is still high. It is necessary to develop a more efficient and low-cost new method for the biocatalytic preparation of NMN.

### SUMMARY

In view of this, an object of the present invention is to provide an enzyme composition for preparing β-nicotinamide mononucleotide (NMN), so that when synthesizing β-nicotinamide mononucleotide with adenosine and nicotinamide as raw materials, 1 molecule of NMN can be generated with only 1 molecule of ATP consumed, and the final step of the reaction to synthesize NMN is irreversible, which can greatly improve the substrate conversion rate and reduce production costs.

Another object of the present invention is to provide a method for synthesizing NMN using the above enzyme composition and to provide related uses of the above enzyme composition in synthesizing NMN.

In order to achieve the above objects, the present invention provides the following technical solutions:
An enzyme composition for preparing β-nicotinamide mononucleotide, comprising purine nucleoside phosphorylase (abbreviated as PNP enzyme) with an EC number of EC 2.4.2.1 and nicotinamide riboside kinase (abbreviated as NRK enzyme) with an EC number of EC 2.7.1.22.

In view of the many defects of the current enzymatic catalysis method, the present invention employs enzymatic reaction technology to provide an alternative process for synthesizing β-nicotinamide mononucleotide using adenosine and nicotinamide. The reaction process is as follows:

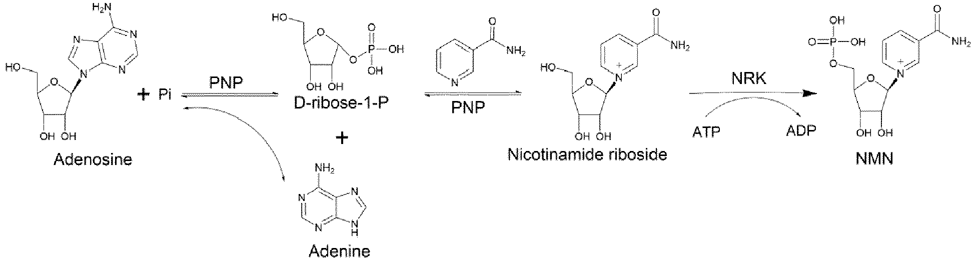

Adenosine and phosphate first synthesize D-ribose-1-phosphate under the action of PNP enzyme, then continue to synthesize nicotinamide ribose with nicotinamide under the catalysis of this enzyme, and then consumes one molecule of ATP for an irreversible reaction to generate NMN under the catalysis of NRK enzyme.

In the above reaction process, the PNP enzyme is a purine nucleoside phosphorylase derived from the group consisting of calf spleen, Bos taurus, Escherichia coli, Salmonella typhimurium, Bacillus cereus, Bacillus clausii, Aeromonas hydrophila, Bovine Salmonella enterica, Bacteroides fragilis, Deinococcus radiodurans, Aeromonas hydrophila, and a combination thereof. Preferably, the PNP enzyme is a purine nucleoside phosphorylase derived from the group consisting of calf spleen, Bos taurus (Bovine), Escherichia coli K12, Salmonella typhimurium (strain ATCC 700720), Bacillus cereus (strain ATCC 14579), Bacillus clausii (strain KSM-K16), Aeromonas hydrophila, Bovine Salmonella enterica, Bacteroides fragilis (strain ATCC 25285), Deinococcus radiodurans (strain ATCC 13939), Aeromonas hydrophila, and a combination thereof.

The present invention requires the participation of PNP enzyme to generate D-ribose-1-phosphate and nicotinamide ribose. The process of generating D-ribose-1-phosphate does not limit the source of PNP enzyme, as long as it belongs to PNP enzyme of EC 2.4.2.1. The process of generating nicotinamide ribose requires specific PNP enzyme to be completed, and the PNP enzymes provided in the present invention are able to complete the two-step reaction.

The NRK enzyme is derived from the group consisting of B. pseudomallei, Ashbya gossypii, Haemophilus influenzae, Saccharomyces cerevisiae, Schizosaccharomyces pombe, Salmonella typhimurium, Cupriavidus metallidurans, Xanthomonas campestris pv. Campestris, Agrobacterium vitis, Pseudarthrobacter chlorophenolicus, Actinobacillus succinogenes, Homo sapiens, Saccharomyces cerevisiae, Ashbya gossypii, and a combination thereof. Preferably, the NRK enzyme is derived from the group consisting of B. pseudomallei K96243, Ashbya gossypii ATCC 10895, Haemophilus influenzae ATCC 51907, Saccharomyces cerevisiae ATCC 204508, Schizosaccharomyces pombe ATCC 24843, Salmonella typhimurium (strain ATCC 700720), Cupriavidus metallidurans (strain ATCC 43123), Deinococcus radiodurans (strain ATCC 13939), Xanthomonas campestris pv. Campestris (strain ATCC 33913), Agrobacterium vitis (strain ATCC BAA-846) (Rhizobium vitis (strain S4)), Pseudarthrobacter chlorophenolicus, Actinobacillus succinogenes (strain ATCC 55618), Homo sapiens (Human), Saccharomyces cerevisiae (strain ATCC 204508) (Baker's yeast), Ashbya gossypii (strain ATCC 10895), and a combination thereof.

The enzyme composition for preparing β-nicotinamide mononucleotide provided by the present invention uses adenosine and nicotinamide as raw materials to synthesize NMN by PNP enzyme and NRK enzyme. Only two enzymes are required to obtain NMN. The raw materials of adenosine and nicotinamide are low in cost, and the by-product adenine can be recycled and reused, significantly reducing costs. In addition, only one molecule of ATP is consumed to generate one molecule of NMN, which greatly reduces the cost. What is even more different from the conventional enzyme catalysis method is that the final reaction of the synthesis of NMN catalyzed by NRK enzyme is irreversible, which can greatly increase the substrate conversion rate and further reduce production costs.

Therefore, the present invention also provides use of the enzyme composition in the manufacture of an enzyme preparation for synthesizing β-nicotinamide mononucleotide or in the synthesis of β-nicotinamide mononucleotide.

Depending on the use, the present invention also provides a method for synthesizing β-nicotinamide mononucleotide by enzymatic catalysis, comprising using adenosine, nicotinamide, ATP, an ion selected from the group consisting of Mg ion and Mn ion, and phosphate as raw materials, and synthesizing β-nicotinamide mononucleotide and phosphate under enzymatic catalysis of the enzyme composition of the present invention. The phosphate can be recycled and used in the next reaction process. The reaction can generally be carried out in a buffer, such as Tris or PBS buffer. For convenient and efficient reaction, PBS buffer can be used as both the reaction medium and the phosphate in the reaction to start the reaction.

Preferably, magnesium ions or manganese ions as coenzyme factors can be provided by one or more of magnesium chloride, magnesium sulfate, magnesium sulfite, magnesium nitrate, manganese chloride, manganese sulfate, and manganese nitrate.

Preferably, the enzyme composition participates in the enzymatic catalysis in the form of a host cell expressing each enzyme, enzyme liquid of each enzyme, or immobilized enzyme of each enzyme. In a specific embodiment of the present invention, the host cell expressing each enzyme is Escherichia coli comprising a vector expressing each enzyme. The vector can separately express one of the two enzymes or co-express the two enzymes. The specific preparation process is as follows:

The strain DNA is extracted as a template, and the DNA fragment of the target PNP enzyme or NRK enzyme is amplified by PCR, which can be optimized according to the codon preference of E. coli. Table 1 of the present invention lists the sequences of the PNP enzyme or NRK enzyme used in specific examples. The amplified gene is digested based on the restriction site, and linked to a vector plasmid digested with the same enzyme. The plasmid with correct sequence verified by sequencing is transformed into the E. coli BL21 (DE3) strain, cultured in LB culture medium, and then induced to expression under IPTG conditions to collect wet cells of each enzyme. The vector plasmid is the commercially available pET28a plasmid, and the amplification primers for each enzyme can be designed according to the coding sequence of each enzyme.

In a specific embodiment of the present invention, the enzyme liquid of each enzyme is an enzyme liquid extracted from a host cell expressing each enzyme. The method is to crush the collected wet cells under high pressure and centrifuge at high speed to collect the supernatant containing crude protein. The liquid is an enzyme liquid containing enzyme, and the supernatant can also be further purified.

Preferably, the method of the present invention also comprises a purification step of β-nicotinamide mononucleotide.

After filtration, separation by chromatography, concentration for crystallization and drying, pure β-nicotinamide mononucleotide is obtained.

The method for generating NMN of the present invention has a substrate conversion rate (calculated as adenosine, a molar ratio of product to adenosine) of 90-99%, and an NMN purity of more than 99%.

It can be seen from the above technical solution that the present invention uses adenosine and nicotinamide as raw materials to generate D-ribose-1-phosphate and nicotinamide ribose intermediate under the enzymatic catalysis of the enzyme composition of PNP enzyme and NRK enzyme, and finally obtains NMN. The entire reaction system only requires the participation of two enzymes, and the by-product adenine can be recycled and reused. In addition, only one molecule of ATP is consumed to generate one molecule of NMN, which greatly reduces the process cost. The final reaction of the synthesis of NMN catalyzed by NRK enzyme is irreversible, which can greatly increase the substrate conversion rate and further reduce production costs.

### DETAILED DESCRIPTION

The present invention discloses an enzyme composition for preparing β-nicotinamide mononucleotide and uses thereof. Those skilled in the art can learn from the content of the present invention and appropriately improve the process parameters for achievement. It should be noted that all similar replacements and modifications are obvious to those skilled in the art, and they are deemed to be included in the present invention. The enzyme composition and use thereof of the present invention have been described through preferred examples. Those skilled in the art can obviously make modifications or appropriate changes and combinations to the enzyme composition and use thereof of the present invention without departing from the content, spirit and scope of the present invention to implement and apply the technology of the present invention.

The synthesis method of the present invention and the schematic diagram of its reaction principle are intended to clearly describe the core reaction route, and do not limit whether the entire reaction can be carried out in one step or in multiple steps.

Each enzyme used in the present invention can be artificially synthesized according to the sequence, or can be induced to expression with a plasmid vector according to the method provided by the present invention to load expression gene of each enzyme and induce expression with the help of host cells.

When performing immobilization, the conventional preparation methods of immobilized enzymes in this field can be referred to. In a specific embodiment of the present invention, the present invention uses LX-1000EP epoxy resin (Sunresin, Xi'an) for one-time mixing and immobilization. The immobilization method is as follows: adding 100 ml of the above PNP pure enzyme liquid and 10 ml of NRK pure enzyme liquid to 1 L of potassium phosphate buffer (1M pH 7.5), mixing well, adding 80 g of LX-1000EP epoxy resin, stirring at room temperature for 8 h, filtering to obtain the resin, washing three times with 25 mM pH 8.0 potassium phosphate buffer, and performing suction filtration to obtain the immobilized enzyme. The immobilized enzyme was measured to have a PNP enzyme activity of 800 U/g and an NRK enzyme activity of 80 U/g.

According to the reaction route of the process of the present invention, the amount of each reaction substance can be adjusted based on the actual situation. For maximized efficiency, the present invention provides the following concentrations and amounts of each reaction substance:

The ratio of PNP enzyme to NRK enzyme is calculated as enzyme activity. The enzyme activity ratio of PNP:NRK is 1: (0.01-100), preferably 1: (0.1-10). The substrate concentration of adenosine is 1-150 mM, the substrate concentration of nicotinamide is 1-150 mM, the substrate concentration of ATP is 1 mM-150 mM, the concentration of magnesium ion or manganese ion is 5 mM-200 mM, and the concentration of phosphate salt or Tris is 5-200 mM.

During the reaction process, the pH of the reaction solution is maintained 6.5-9.5, preferably 7.0-8.5; and the reaction temperature is 25-50°C, preferably 35-45°C.

For relevant information on enzymes used in specific examples of the present invention, see Table 1 and Table 2.

**Table 1**

| PNP enzyme sequence listing | | | |
|---|---|---|---|
| corresponding example | Uniprot ID | Source | Gene sequence |
| Example 1 | Q68719 | *Bos taurus (Bovine)* | MANGYTYEDYQDTAKWLLSHTEQRPQVA VICGSGLGGLVNKLTQAQTFDYSEIPNFPE STVPGHAGRLVFGILNGRACVMMQGRFH MYEGYPFWKVTFPVRVFRLLGVETLVVTN AAGGLNPNFEVGDIMLIRDHINLPGFSGEN PLRGPNEERFGVRFPAMSDAYDRDMRQK AHSTWKQMGEQRELQEGTYVMLGGPNFE TVAECRLLRNLGADAEGMSTVPEVIVARH CGLRVFGFSLITNKVIMDYESQGKANHEE VLEAGKQAAQKLEQFVSLLMASIPVSGHT G as shown in SEQ ID NO. 1 |
| Example 2 | P45563 | *Escherichia coli (strain K12)* | as shown in SEQ ID NO. 2 |
| Example 2 | Q5WA Q8 | Bacillus clausii (strain KSM-K16) | MTEFKQTPHINAKGEIAETVLLPGDPLRAK FIAERFLTDVVQFNSVRNMFGFTGTYNGK RISVMGTGMGMPSMGIYSYELIHVFGVKN LIRVGSCGALQDDLNLYDVILGMGASTNS NYVNQYHLPGTFAPLASYSLLEKAKKAAD EKGVPVHVGNILSSDTFYNADEDAAGKW RDMGILGIEMEAAALYMNAAQAGVNALC ILTVSDHIFKEEETTAEERETSFTNMMEIAL ELA as shown in SEQ ID NO. 3 |
| Example 3 | Q5LG7 δ | Bacteraides fragilis(strai n ATCC 25285 ) | MKKYFPSSELIINEDGSVFHLHVKPEWLAD KVILVGDPGRVALVASHFENKECEVESREF KTVTGTYKGKRITVVSTGIGCDNIDIVVNE LDALANIDFQTREEKEHLRSLELVRIGTCG GLQPNTPVGTFVCSEKSIGFDGLLNFYAGR NAVCDLPFERAFLNHMGWSGNMCAPAPY VIDANAELIDRIAQEDMVRGVTIAAGGFFG PQGRELRVPLADPKQNDKIEKFEYKGYKIT NFEMESSALAGLSKLMGHKAMTVCMVIA NRLIKEANTGYKNTIDTLIKTILDRI as shown in SEQ ID NO. 4 |
| Example 4 | Q8ZJV 7 | Salmonella typhimurium (ATCC 700720) | MATPHINAEMGDFADVVLMPGDPLRAKHI AETFLENVREVNNVRGMLGFTGTYKGRKI SVMGHGMGIPSCSIYTKELITDFGVKKIIRV GSCGAVRMDVKLRDVVIGMGACTDSKVN RIRFKDHDFAAIADFDMVRNAVDAAKAL GVDARVGNLFSADLFYSPDGEMFDVMEK YGVLGVEMEAAGIYGVAAEFGAKALTICT VSDHIRTHEQTTAAERQTTFNDMIKIALES VLLGDKE as shown in SEQ ID NO. 5 |
| Example 5 | A0A4P 7IUL0 | Aeromonas hydrophila | MQQDPVLNAVATILARKPGFAPRAAMILG SGLGVLADGLEEKVTIPYEELDGFPVSTVA GHSGELVLGKLHGVDVVCMKGRGHYYE HETMKVMTTPVRTFKRLGCELLLVTNAA GSLRPERIGVGSLVIFNDHINTMPGTPMTG ANDEAYGPRFFSLANAYDKALRGEALALA EREGIAVNQGVFVSYSGPCFETAAEIRMM QIIGGDVVGMSVVPEVVSAAHCALPVLAI CAITNMAEGLGDVQLSHEQTLKCAKLAET DFIRLINAFIKHHFQ as shown in SEQ ID NO. 6 |
| Example 6 | Q9RSF 8 | Deinococcus radiodurans (strain | MDIGELFGLAHVHEQRPLLHQLLPFGGGD FFHGGSIANPTEKKAGRAANRRKFGRNAL RSPDTLRGMSIHLNAEKGQIAETVLLPGDP LRARHIAETFLENPVQHNTVRGMLGFTGT YKGERVSVQGTGMGIPSCMIYVNELIQDY |
| | | ATCC 13939) | GCKNLIRVGTAGSYQEGVHVRDLVLAQA ACTDSNINRVRFGERTFAPIADFELLLRAY QIAQERGFSAHVGNIMSSDTFYNDNPTEFQ RWAEFGVLAVEMEAAGLYTLAAKYGVR ALTILTISDHLVTHEVTSAEERQLTFNGMIE VALDAALGLEKKA as shown in SEQ ID NO. 7 |
| Example 6 | LACC1 | *Homo sapiens (Human)* | MENEFTYEDYQRTAEWLRSHTKHRPQVA VICGSGLGGLTAKLTQPQAFDYNEIPNFPQ STVQGHAGRLVFGFLNGRSCVMMQGRFH MYEGYSLSKVTFPVRVFHLLGVDTLVVTN AAGGLNPKFEVGDIMLIRDHINLPGFCGQN PLRGPNDERFGVRFPAMSDAYDRDMRQK AFNAWKQMGEQRELQEGTYIMSAGPTFET VAESCLLRMLGADAVGMSTVPEVIVARHC GLRVFGFSLITNKVVMDYNNLEKASHQEV LEAGKAAAQKLEQFVSILMESIPPRERAN as shown in SEQ ID NO. 8 |

**Table 2**

| NRK enzyme sequence listing | | | |
|---|---|---|---|
| | UniprotI D | Source | Gene sequence |
| Example 1 | P44308 | Haemophilus influenzae ATCC 51907 | MGFTTGREFHPALRMRAKYNAKYLGT KSEREKYFHLAYNKHTQFLRYQEQIMS KTKEKKVGVIFGKFYPVHTGHINMIYE AFSKVDELHVIVCSDTVRDLKLFYDSK MKRMPTVQDRLRWMQQIFKYQKNQIFI HHLVEDGIPSYPNGWQSWSEAVKTLFH EKHFEPSIVFSSEPQDKAPYEKYLGLEV SLVDPDRTFFNVSATKIRTTPFQYWKFI PKEARPFFAKTVAILGGESSGKSVLVNK LAAVFNTTSAWEYGREFVFEKLGGDEQ AMQYSDYPQMALGHQRYIDYAVRHSH KIAFIDTDFITTQAFCIQYEGKAHPFLDS MIKEYPFDVTILLKNNTEWVDDGLRSL GSQKQRQQFQQLLKKLLDKYKVPYIEIE SPSYLDRYNQVKAVIEKVLNEEEISELQ NTTFPIKGTSQ as shown in SEQ ID NO. 9 |
| Example 2 | P24518 | Salmonella typhimunum( strain ATCC | MSSFDYLKTAIKQQGCTLQQVADASG MTKGYLSQLLNAKIKSPSAQKLEALHR FLGLEFPRRQKNIGVVFGKFYPLHTGHI YLIQRACSQVDELHIIMGYDDTRDRGLF EDSAMSQQPTVSDRLRWLLQTFKYQK |
| | | 700720) | NIRIHAFNEEGMEPYPHGWDVWSNGIK AFMAEKGIQPSWIYTSEEADAPQYLEHL GIETVLVDPERTFMNISGAQIRENPFRY WEYIPTEVKPFFVRTVAILGGESSGKST LVNKLANIFNTTSAWEYGRDYVFSHLG GDEMALQYSDYDKIALGHAQYIDFAVK YANKVAFIDTDFVTTQAFCKKYEGREH PFVQALIDEYRFDLVILLENNTPWVADG LRSLGSSVDRKAFQNLLVEMLKENNIEF VHVKEADYDGRFLRCVELVKEMMGEQ G as shown in SEQ ID NO. 10 |
| Examples 3, 4 | Q63W44 | B.pseudomall ei K96243 | MPEPGVGAHAGGLDADAALARFFVITG GPGSGKSTLIDALEARGYARSHEAGRG VIQDQVAIGGHALPWRDRAAFADMML GWEMRSHHLARRARGPVFFDRGVPDVI GYLKLSALPVPAHLVAAAERFRYRRDV FIAPPWPEIYAQDAERRQDYAEAVRTY GAMVDTYAACGYRLIELPRASVDERCR FVLDVVEAAAAV as shown in SEQ ID NO. 11 |
| Example 5 | P53915 | Saccharomyce s cerevisiae ATCC 204508 | MTSKKVILVALSGCSSSGKTTIAKLTAS LFTKATLIHEDDFYKHDNEVPVDAKYN IQNWDSPEALDFKLFGKELDVIKQTGKI ATKLIHNNNVDDPFTKFHIDRQVWDEL KAKYDSINDDKYEVVIVDGFMIFNNTGI SKKFDLKILVRAPYEVLKKRRASRKGY QTLDSFWVDPPYYFDEFVYESYRANHA QLFVNGDVEGLLDPRKSKNIKEFINDDD TPIAKPLSWVCQEILKLCKD as shown in SEQ ID NO. 12 |
| Example 6 | P62511 | Ashbya gossypii (strain ATCC 10895) | MTSQLAGFKGTRGTLLVGIGGCSSSGKS TIAKLAVQVLEDAVLVHQDDFYRHDDE VPFDEEYQIGNWDVPEALDMAQFEREL DHIRATGRPAAKLVHNGNIDDVGKFGIS EEYLEELRRRYRGRISQPVVLVDGFML YHDDKVAARFDCRLLVRAPYATMKAR RASRGGYKTLDSFWQDPPFYFDKFVYK SYAATHARLFRNCDVEDRLVAPDVQEI YNGDEAQITCVLEQVLDAIAAAQC as shown in SEQ ID NO. 13 |

The present invention will be further described below in conjunction with the examples.

### Example 1:

### Source of PNP enzyme: Bos taurus (Bovine)

### Preparation source of NRK enzyme: Haemophilus influenzae (ATCC 51907)

PNP/NRK enzyme ratio 1:1, 37°C, pH 6.5. The substrate concentration of adenosine was 150 mM, the substrate concentration of nicotinamide was 150 mM, the substrate concentration of ATP was 150 mM, the concentration of magnesium ions was 20 mM, the concentration of phosphate salt was 20 mM, and the substrate conversion rate was 90%.

Preparation of PNP enzyme: The protein sequence of PNP enzyme derived from Bos taurus is shown in Table 1. The corresponding DNA gene sequence was synthesized in vitro by General Biological (AnHui) Co. Ltd. after codon optimization, digested with Nde I/Xho I (NEB Company), and linked to the pET28a plasmid (purchased from Addgene) digested with the same enzyme. The constructed plasmid was transformed into the E. coli BL (DE3) strain (Shanghai Weidi Biotech). The confirmed correct colony was inoculated into LB culture medium containing 100 µM kanamycin, wherein the LB medium comprised of 1% tryptone, 0.5% yeast powder, 1% NaCl, 1% dipotassium hydrogen phosphate, 1% potassium dihydrogen phosphate and 5% glycerol. When the cells grew to mid-to-late logarithmic phase, 0.2 mM isopropyl-β-D-thiogalactopyranoside (IPTG) was add to induce protein expression at 30°C for 5 h, and then the wet cells were collected by centrifugation.

Preparation of NRK enzyme: The DNA of Haemophilus influenzae ATCC 51907 strain was extracted as a template to amplify the NadR gene fragment (genbank: NZ_CP009610.1) by PCR, wherein the NadR forward primer was F: 5'-catatgcgagctaagtataacgcaaaat-3', and the NadR reverse primer was R: 5'-ctcgagtcattgagatgtcccttttataggaaaggt-3', synthesized by Genewiz. The protein sequence corresponding to this gene fragment is shown in Table 1. The amplified gene was digested with Nde I/Xho I purchased from NEB Company and linked to the pET28a plasmid (purchased from Addgene) digested with the same enzyme. The constructed plasmid was trasnformed into E.coli BL21 (DE3) strain (Shanghai Weidi Biotech). The confirmed correct colony was inoculated into LB culture medium containing 100 µM kanamycin, wherein the LB medium comprised 1% tryptone, 0.5% yeast powder, 1% NaCl, 1% dipotassium hydrogen phosphate, 1% potassium dihydrogen phosphate and 5% glycerol. When the cells grew to mid-to-late logarithmic phase, 0.2 mM isopropyl-β-D-thiogalactopyranoside (IPTG) was add to induce protein expression at 30°C for 5 h, and then the wet cells were collected by centrifugation.

100 g of wet cells of each of the above PNP enzyme and NRK enzyme was weighed, resuspended in 1000 ml of 20 mM, pH 7.5 Tris aqueous solution and crushed under high pressure to obtain crude enzyme liquids of PNP enzyme and NRK enzyme. The well-known methods for measuring the enzyme activity of PNP enzyme and NRK enzyme in the prior art were used, and the amount of enzyme required to convert 1 micromole of substrate within 1 minute under specific conditions is regarded as one activity unit (U). The enzyme activity of the PNP enzyme of the present invention is defined as: the amount of enzyme required to convert 1 micromole of adenosine into D-ribose-1-phosphate in 1 minute is one activity unit (U). The enzyme activity of the NRK enzyme of the present invention is defined as: the amount of enzyme required to convert 1 micromole of nicotinamide ribose into β-nicotinamide ribose mononucleotide in one minute is one activity unit (U). The enzyme activity of the above PNP crude enzyme liquid was measured to be 75 U/ml, and the NRK enzyme activity was 600 U/ml.

150 mM adenosine, 150 mM nicotinamide, 20 mM MgCl₂, 150 mM ATP, and 20 mM pH8.0 PBS buffer were added in a 1 L reactor. The pH value was adjusted to 6.5, and the total volume of the reaction solution was 0.8 L. 32 ml of the above PNP crude enzyme liquid and 4 ml of the above NRK crude enzyme liquid were added and stirred at 37°C for 8 h while maintaining pH 8.0 to generate 135 mM (45.1 g/L) NMN, with a substrate conversion rate of 90%. The reaction solution was filtered, separated by chromatography, concentrated for crystallization, and dried to obtain 27.1 g of pure NMN, with a purity of 99.2% and an NMR result of ¹H NMR (400 MHz, D₂O) δ 9.46 (s, 1H), 9.29 (d, *J* = 5.9 Hz, 1H), 8.99 (d, *J* = 7.9 Hz, 1H), 8.31 (t, *J* = 7.1 Hz, 1H), 6.22 (d, *J* = 5.1 Hz, 1H), 4.64 (s, 1H), 4.58-4.55 (m, 1H), 4.46-4.43 (m, 1H), 4.32-4.29 (m, 1H), 4.17-4.13 (m, 1H).

### Example 2:

Source of PNP enzyme 1: *Escherichia coli (strain K12)*
Source of PNP enzyme 2: Bacillus clausii (strain KSM-K16)
Source of NRK enzyme: Salmonella typhimurium (ATCC 700720)

PNP/NRK enzyme ratio 100:1, 35°C, pH 8.5. The substrate concentration of adenosine was 135 mM, the substrate concentration of nicotinamide was 135 mM, the substrate concentration of ATP was 135 mM, the concentration of magnesium ions was 200 mM, the concentration of Tris was 20 mM, and the substrate conversion rate was 92%.

Preparation of PNP enzyme 1: The protein sequence of PNP enzyme derived from Escherichia coli (strain K12) is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation.

Preparation of PNP enzyme 2: The protein sequence of PNP enzyme derived from acillus clausii (strain KSM-K16) is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation.

Preparation of NRK enzyme: The protein sequence of NRK enzyme derived from Salmonella typhimurium (ATCC 700720) is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation.

100 g of wet cells of each of the above PNP enzyme 1, PNP enzyme 2 and NRK enzyme was weighed, resuspended in 1000 ml of 20 mM, pH 7.5 Tris aqueous solution and crushed under high pressure to obtain crude enzyme liquid of PNP enzyme 1, PNP enzyme 2 and NRK enzyme. The enzyme activity of the crude enzyme liquid of PNP enzyme 1 was measured to be 200 U/ml, the enzyme activity of the crude enzyme liquid of PNP enzyme 2 was measured to be 500 U/ml and the NRK enzyme activity was 50 U/ml.

135 mM adenosine, 135 mM nicotinamide, 200 mM MgCl₂, 135 mM ATP, and 20 mM Tris were added in a 1 L reactor. The pH value was adjusted to 8.5, and the total volume of the reaction solution was 0.8 L. 50 ml of the crude enzyme liquid of PNP enzyme 1, 30 ml of the crude enzyme liquid of PNP enzyme 2 and 5 ml of the NRK crude enzyme liquid were added and stirred at 35°C for 24 h while maintaining pH 8.5 to generate 124.2 mM (45.1 g/L) NMN, with a substrate conversion rate of 92%. The reaction solution was filtered, separated by chromatography, concentrated for crystallization, and dried to obtain 28.2 g of pure NMN, with a purity of 99.3%.

### Example 3:

Source of PNP enzyme: Bacteroides fragilis (strain ATCC 25285)
Source of NRK enzyme: *B. pseudomallei K96243*
Immobilized enzyme form;
the ratio of PNP to NRK enzyme was 10:1.

PNP/NRK enzyme ratio 10:1, 40°C, pH 7.0. The substrate concentration of adenosine was 50 mM, the substrate concentration of nicotinamide was 50 mM, the substrate concentration of ATP was 80 mM, the concentration of manganese ions was 5 mM, the concentration of phosphate salt was 200 mM, and the substrate conversion rate was 91.1%.

Preparation of PNP enzyme: The protein sequence of PNP enzyme derived from Bacteroides fragilis (strain ATCC 25285) is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation.

Preparation of NRK enzyme: The protein sequence of NRK enzyme derived from B. pseudomallei K96243 is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation.

The collected bacterial cells containing PNP enzyme and NRK enzyme were mixed, crushed under high pressure, and centrifuged to collect the supernatant containing crude protein. The supernatant was purified using a nickel ion chelating affinity column (Yisheng Biotechnology) to obtain PNP pure enzyme liquid and NRK pure enzyme liquid. The enzyme activity of PNP pure enzyme liquid was 6800 U/ml, and the enzyme activity of NRK pure enzyme liquid was 6400 U/ml.

The obtained pure enzyme liquid was immobilized once with LX-1000EP epoxy resin (Sunresin, Xi'an), and the method was as follows. 100 ml of the above PNP pure enzyme liquid and 10 ml of NRK pure enzyme liquid were added to 1 L of potassium phosphate buffer (1M pH7.5), mixed well, added with 80 g of LX-1000EP epoxy resin, stirred at room temperature for 8 h, filtered to remove the resin, washed three times with 25 mM pH 8.0 potassium phosphate buffer, and subjected to suction filtration to obtain the immobilized enzyme. The immobilized enzyme was measured to have a PNP enzyme activity of 800 U/g and an NRK enzyme activity of 80 U/g.

50 mM adenosine, 50 mM nicotinamide, 5 mM MnCl₂, 80 mM ATP, and 200 mM pH7.0 PBS buffer were added in a 1 L reactor. The pH value was adjusted to 7.0, and the total volume of the reaction solution was 0.8 L. 20 g of the above immobilized enzyme was added and stirred at 40°C for 8 h while maintaining pH 7.0 to generate 45.5 mM (15.2 g/L) NMN, with a substrate conversion rate of 91.1%. The reaction solution was filtered, separated by chromatography, concentrated for crystallization, and dried to obtain 9.12 g of pure NMN, with a purity of 99.2%.

### Example 4:

Source of PNP enzyme: Salmonella typhimurium (ATCC 700720);
Source of NRK enzyme: *B. pseudomallei K96243* purified enzyme
PNP/NRK enzyme ratio 1:100, 35°C, pH 7.5. The substrate concentration of adenosine was 100 mM, the substrate concentration of nicotinamide was 100 mM, the substrate concentration of ATP was 100 mM, the concentration of magnesium ions was 20 mM, the concentration of phosphate salt was 5 mM, and the substrate conversion rate was 99%.

Preparation of PNP enzyme: The protein sequence of PNP enzyme derived from Salmonella typhimurium (ATCC 700720) is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation. PNP pure enzyme liquid was prepared according to the method of Example 3, and the enzyme activity of PNP pure enzyme liquid was 6200 U/ml.

100 mM adenosine, 100 mM nicotinamide, 20 mM MgCl₂, 100 mM ATP, and 20 mM pH8.0 PBS buffer were added in a 1000 ml Erlenmeyer flask. The pH value was adjusted to 8.0, and the total volume of the reaction solution was 500 ml. 4.8 ml of the above PNP pure enzyme liquid and 46 ml of NRK pure enzyme liquid derived from *B. pseudomallei K96243* were added and stirred at 35°C for 24 h while maintaining pH 8.0 to generate 99 mM (30.07 g/L) NMN, with a substrate conversion rate of 99%. The reaction solution was filtered, separated by chromatography, concentrated for crystallization, and dried to obtain 12.7 g of pure NMN, with a purity of 99.4%.

### Example 5:

Source of PNP enzyme: Aeromonas hydrophila
Source of NRK enzyme: Saccharomyces cerevisiae ATCC 204508
Co-expression of NRK and PNP + whole cell catalytic form (enzyme activity ratio 1:1)
PNP/NRK enzyme ratio 1:1, 37°C, pH 8.0. The substrate concentration of adenosine was 135 mM, the substrate concentration of nicotinamide was 135 mM, the substrate concentration of ATP was 135 mM, the concentration of magnesium ions was 20mM, the concentration of phosphate salt was 20 mM, and the substrate conversion rate was 92%.

Whole cell preparation: The protein sequence of PNP enzyme derived from Aeromonas hydrophila is shown in Table 1. The corresponding DNA gene sequence was synthesized in vitro by General Biological (AnHui) Co. Ltd. after codon optimization, and digested with BamH I/Not I (NEB Company). The protein sequence of NRK enzyme derived from Saccharomyces cerevisiae ATCC 204508 is shown in Table 1. The corresponding DNA gene sequence was synthesized in vitro by General Biological (AnHui) Co. Ltd. after codon optimization, and digested with BamH I/Not I (NEB Company). Both of the two genes digested above were linked to the pRSFDuet-1 plasmid (purchased from Biofeng) digested with the same enzyme. The constructed plasmid was transformed into the E. coli BL21 (DE3) strain (Shanghai Weidi Biotech). The confirmed correct colony was inoculated into LB culture medium containing 100 µM kanamycin, wherein the LB medium comprised 1% tryptone, 0.5% yeast powder, 1% NaCl, 1% dipotassium hydrogen phosphate, 1% potassium dihydrogen phosphate and 5% glycerol. When the cells grew to mid-to-late logarithmic phase, 0.2 mM isopropyl-β-D-thiogalactopyranoside (IPTG) was add to induce protein expression at 30°C for 5 h, and then the wet cells were collected by centrifugation.

The wet cells after centrifugation were washed twice with 20 mM pH 7.0 PBS buffer, resuspended, added with 0.15% Triton X-100, stirred at low speed for 30 min, and then centrifuged to collect wet cells. The wet cells after permeabilization were measured to have a PNP enzyme activity of 750 U/g and an NRK enzyme activity of 750 U/g.

135 mM adenosine, 135 mM nicotinamide, 20 mM MgCl₂, 135 mM ATP, and 20 mM pH8.0 PBS buffer were added in a 1 L reactor. The pH value was adjusted to 8.0, and the total volume of the reaction solution was 0.8 L. 32 g of the above wet cells was added and stirred at 37°C for 12 h while maintaining pH 8.0 to generate 124.2 mM (41.48 g/L) of NMN, with a substrate conversion rate of 92%. The reaction solution was filtered, separated by chromatography, concentrated for crystallization, and dried to obtain 24.22 g of pure NMN, with a purity of 99.1%.

### Example 6:

Source of PNP enzyme 1: Deinococcus radiodurans (strain ATCC 13939)
Source of PNP enzyme 2: Homo sapiens (Human)
Source of NRK enzyme: Ashbya gossypii (strain ATCC 10895)

In this example, the effects of different forms of crude enzyme, pure enzyme, and immobilized enzyme with the same enzyme activity (1:0.518) were compared.

PNP enzyme 1/NRK enzyme ratio 1:0.518, crude enzyme, 37°C, pH 8.0. The substrate concentration of adenosine was 100 mM, the substrate concentration of nicotinamide was 100 mM, the substrate concentration of ATP was 100 mM, the concentration of magnesium ions was 20 mM, the concentration of phosphate salt was 20 mM, and the substrate conversion rate was 90%.

PNP enzyme 1/NRK enzyme ratio 1:0.518, pure enzyme, 37°C, pH 8.0. The substrate concentration of adenosine was 100 mM, the substrate concentration of nicotinamide was 100 mM, the substrate concentration of ATP was 100 mM, the concentration of magnesium ions was 20 mM, the concentration of phosphate salt was 20 mM, and the substrate conversion rate was 95%.

PNP enzyme 1/NRK enzyme ratio 1:0.518, immobilized enzyme, 37°C, pH 8.0. The substrate concentration of adenosine was 100 mM, the substrate concentration of nicotinamide was 100 mM, the substrate concentration of ATP was 100 mM, the concentration of magnesium ions was 20 mM, the concentration of phosphate salt was 20 mM, and the substrate conversion rate was 94%.

PNP enzyme 1/NRK enzyme ratio 1:0.518, immobilized enzyme, 25°C, pH 9.5. The substrate concentration of adenosine was 1 mM, the substrate concentration of nicotinamide was 1 mM, the substrate concentration of ATP was 1 mM, the concentration of magnesium ions was 1 mM, the concentration of phosphate salt was 5 mM, and the substrate conversion rate was 99%.

PNP enzyme 1/NRK enzyme ratio 1:0.518, immobilized enzyme, 45°C, pH 9.5. The substrate concentration of adenosine was 1 mM, the substrate concentration of nicotinamide was 1 mM, the substrate concentration of ATP was 1 mM, the concentration of magnesium ions was 1 mM, the concentration of phosphate salt was 5 mM, and the substrate conversion rate was 99%.

Preparation of PNP enzyme 1: The protein sequence of PNP enzyme derived from Deinococcus radiodurans (strain ATCC 13939) is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation.

Preparation of PNP enzyme 2: The protein sequence of PNP enzyme derived from Homo sapiens (Human) is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation.

Preparation of NRK enzyme: The protein sequence of NRK enzyme derived from Ashbya gossypii (strain ATCC 10895) is shown in Table 1. The corresponding DNA gene sequence was synthesized in vitro by General Biological (AnHui) Co. Ltd. after codon optimization. The construction of genetically recombinant bacteria and bacterial culture were performed according to the method of Example 1, and wet cells were collected by centrifugation.

100 g of the above PNP enzyme 1 wet cells, PNP enzyme 2 wet cells, and NRK enzyme wet cells was weighed respectively, resuspended in 1000 ml of 20 mM, pH 7.5 Tris aqueous solution and crushed under high pressure to obtain crude enzyme liquids of PNP enzyme and NRK enzyme. The enzyme activity of the crude enzyme liquid of PNP enzyme 1 was measured to be 450 U/ml, the enzyme activity of the crude enzyme liquid of PNP enzyme 2 was measured to be 550 U/ml, and the enzyme activity of the crude enzyme liquid of NRK enzyme was 760 U/ml.

The crude enzyme liquids of PNP enzyme 1 and NRK enzyme prepared according to the above method were centrifuged to collect the supernatant. The supernatants were purified using s nickel ion chelate affinity column (Yisheng Biotechnology) to obtain pure enzyme liquids of PNP enzyme 1 and NRK enzyme. The enzyme activity of pure enzyme liquid of PNP enzyme 1 was 4800 U/ml, and the enzyme activity of pure enzyme liquid of NRK enzyme was 6700 U/ml.

The pure enzyme liquids obtained above were immobilized once with LX-1000EP epoxy resin (Sunresin, Xi'an), and the method was as follows. 50 ml of the above pure enzyme liquid of PNP enzyme 1 and 40 ml of NRK pure enzyme liquid were added to 2 L of potassium phosphate buffer (1M pH7.5), mixed well, added with 160 g of LX-1000EP epoxy resin, stirred at room temperature for 8 h, filtered to remove the resin, washed three times with 25 mM pH 8.0 potassium phosphate buffer, and subjected to suction filtration to obtain the immobilized enzyme. The immobilized enzyme was measured to have a PNP enzyme 1 activity of 98.4 U/g and an NRK enzyme activity of 190 U/g.

Crude enzyme form (PNP enzyme 1): 100 mM adenosine, 100 mM nicotinamide, 20 mM MgCl₂, 100 mM ATP, 20 mM pH8.0 PBS buffer were added in a 1L reactor. The pH value was adjusted to 8.0, and the total volume of the reaction solution was 0.8 L. 35 ml of the crude enzyme liquid of PNP enzyme 1 and 40 ml of the NRK crude enzyme liquid were added and stirred at 37°C for 6 h while maintaining pH 8.0 to generate 90 mM (30.06 g/L) of NMN, with a substrate conversion rate of 90%. The reaction solution was filtered, separated by chromatography, concentrated for crystallization, and dried to obtain 18.04 g of pure NMN, with a purity of 99.1%.

Crude enzyme form (PNP enzyme 2): 100 mM adenosine, 100 mM nicotinamide, 20 mM MgCl₂, 100 mM ATP, 20 mM pH8.0 PBS buffer were added in a 1L reactor. The pH value was adjusted to 8.0, and the total volume of the reaction solution was 0.8 L. 28.8 ml of the crude enzyme liquid of PNP enzyme 2 and 40 ml of the NRK crude enzyme liquid prepared above were added and stirred at 37°C for 6 h while maintaining pH 8.0 to generate 1 mM (0.33 g/L) of NMN, with a substrate conversion rate of 1%. The substrate conversion rate was too low for purification to obtain pure product.

The result data of the use of PNP enzyme 2, which can only complete the first step of the reaction but not the second step, showed that the reaction can only proceed to the first step and failed to continue or continued at an extremely low efficiency. This also demonstrates that the process of generating nicotinamide ribose requires a specific PNP enzyme.

Pure enzyme form (PNP enzyme 1): 100 mM adenosine, 100 mM nicotinamide, 20 mM MgCl₂, 100 mM ATP, 20 mM pH8.0 PBS buffer were added in a 1 L reactor. The pH value was adjusted to 8.0, and the total volume of the reaction solution was 0.8 L. 3.28 ml of the pure enzyme liquid of PNP enzyme 1 and 4.53 ml of the NRK pure enzyme liquid were added and stirred at 37°C for 5 h while maintaining pH 8.0 to generate 95 mM (31.73 g/L) of NMN, with a substrate conversion rate of 95%. The reaction solution was filtered, separated by chromatography, concentrated for crystallization, and dried to obtain 20.05 g of pure NMN, with a purity of 99.4%.

Immobilized enzyme form (PNP enzyme 1): 100 mM adenosine, 100 mM nicotinamide, 20 mM MgCl₂, 100 mM ATP, 20 mM pH8.0 PBS buffer were added in a 1 L reactor. The pH value was adjusted to 8.0, and the total volume of the reaction solution was 0.8 L. 160 g of the immobilized enzyme of PNP enzyme 1 and NRK enzyme was added and stirred at 37°C for 5.5 h while maintaining pH 8.0 to generate 94 mM (31.39 g/L) of NMN, with a substrate conversion rate of 94%. The reaction solution was filtered, separated by chromatography, concentrated for crystallization, and dried to obtain 19.8 g of pure NMN, with a purity of 99.4%.

Immobilized enzyme form (PNP enzyme 1, 25°C): 1 mM adenosine, 1 mM nicotinamide, 5 mM MgCl₂, 1 mM ATP, 5 mM pH8.0 PBS buffer were added in a 1 L reactor. The pH value was adjusted to 9.5, and the total volume of the reaction solution was 0.8 L. 160 g of the immobilized enzyme of PNP enzyme 1 and NRK enzyme was added and stirred at 25°C for 5.5 h while maintaining pH 9.5 to generate 0.99 mM (0.33 g/L) of NMN, with a substrate conversion rate of 99%. The reaction solution was filtered, separated by chromatography, concentrated for crystallization, and dried to obtain 0.13 g of pure NMN, with a purity of 99.4%.

Immobilized enzyme form (PNP enzyme 1, 45°C): 1 mM adenosine, 1 mM nicotinamide, 5 mM MgCl₂, 1 mM ATP, 5 mM pH8.0 PBS buffer were added in a 1 L reactor. The pH value was adjusted to 9.5, and the total volume of the reaction solution was 0.8 L. 160 g of the immobilized enzyme of PNP enzyme 1 and NRK enzyme was added and stirred at 45°C for 0.5 h while maintaining pH 9.5 to generate 0.99 mM (0.33 g/L) of NMN, with a substrate conversion rate of 97%. The reaction solution was filtered, separated by chromatography, concentrated for crystallization, and dried to obtain 0.13 g of pure NMN, with a purity of 99.4%.

The above are only preferred embodiments of the present invention. It should be noted that those skilled in the art can make several improvements and modifications without departing from the principles of the present invention, and these improvements and modifications should be regarded as the protection scope of the present invention.

## Claims

1. An enzyme composition for preparing β-nicotinamide mononucleotide, consisting of purine nucleoside phosphorylase with an EC number of EC 2.4.2.1 and nicotinamide riboside kinase with an EC number of EC 2.7.1.22; wherein, the purine nucleoside phosphorylase is a purine nucleoside phosphorylase derived from the group consisting of calf spleen, Bos taurus, Escherichia coli, Salmonella typhimurium, Bacillus cereus, Bacillus clausii, Aeromonas hydrophila, Bovine Salmonella enterica, Bacteroides fragilis, Deinococcus radiodurans, Aeromonas hydrophila, and a combination thereof.

2. The enzyme composition according to claim 1, wherein the nicotinamide riboside kinase is derived from the group consisting of B. pseudomallei, Ashbya gossypii, Haemophilus influenzae, Saccharomyces cerevisiae, Schizosaccharomyces pombe, Salmonella typhimurium, Cupriavidus metallidurans, Xanthomonas campestris pv. Campestris, Agrobacterium vitis, Pseudarthrobacter chlorophenolicus, Actinobacillus succinogenes, Homo sapiens, Saccharomyces cerevisiae, Ashbya gossypii, and a combination thereof.

3. Use of the enzyme composition according to any one of claims 1 to 2 in the manufacture of an enzyme preparation for synthesizing β-nicotinamide mononucleotide or in the synthesis of β-nicotinamide mononucleotide.

4. A method for synthesizing β-nicotinamide mononucleotide by enzymatic catalysis, comprising using adenosine, nicotinamide, ATP, an ion selected from the group consisting of Mg ion and Mn ion, and phosphate as raw materials, and synthesizing β-nicotinamide mononucleotide under enzymatic catalysis of the enzyme composition according to any one of claims 1 to 2.

5. The method according to claim 4, wherein the enzyme composition participates in the enzymatic catalysis in the form of a host cell expressing each enzyme, enzyme liquid of each enzyme, or immobilized enzyme of each enzyme.

6. The method according to claim 5, wherein the host cell expressing each enzyme is Escherichia coli comprising a vector expressing each enzyme or a vector co-expressing two enzymes.

7. The method according to claim 5, wherein the enzyme liquid of each enzyme is an enzyme liquid extracted from a host cell expressing each enzyme.

8. The method according to claim 4, further comprising a step of purifying β-nicotinamide mononucleotide, consisting of
filtering, separating by chromatography, concentrating for crystallization, and drying to obtain pure β-nicotinamide mononucleotide.
